Europäisches Patentamt

European Patent Office

Office européen des brevets

(19)

(11) Publication number: **0 114 577**
**A1**

(12) **EUROPEAN PATENT APPLICATION**

(21) Application number: **83830007.7**

(22) Date of filing: **21.01.83**

(51) Int. Cl.³: **A 61 K 9/50**

(43) Date of publication of application:
**01.08.84 Bulletin 84/31**

(84) Designated Contracting States:
**AT BE CH DE FR GB LI NL SE**

(71) Applicant: **MAGIS FARMACEUTICI S.R.L.**
**Via Cacciamali n. 34/36/38 Zona Industriale - Loc. Noce**
**I-25100 Brescia(IT)**

(72) Inventor: **Moroni, Adolfo**
**Via Taramelli, 7**
**Brescia(IT)**

(74) Representative: **Zorzoli, Franco**
**c/o BUGNION S.p.A. Via Carlo Farini 81**
**I-20159 Milan(IT)**

(54) New pharmaceutical composition.

(57) A new pharmaceutical composition for oral administration of cytidine diphosphate choline ester utilizes liposomes as carrier. In that way hematic levels are obtained which are comparable with those obtained by intravenous administration while taking advantage of an administration way which is more comfortable and more acceptable to patients.

EP 0 114 577 A1

Croydon Printing Company Ltd.

New pharmaceutical composition

The present invention relates to new pharmaceutical compositions for oral administration of cytidine diphosphate choline ester, hereinafter called CDPC for the sake of brevity, and to their preparation.

More particularly, the present invention relates to new pharmaceutical compositions for oral administration of CDPC or its salts, characterized in that the active ingredient is carried by means of liposomes.

CDPC is an active ingredient which usually is parenterally administered, in aqueous solutions having concentrations ranging from 50 to 250 mg/ml. As a matter of fact the oral administration, with hitherto known pharmaceutical compositions, met with a well determinate limit as it was not possible to reach hematic levels high enough to show an appreciable therapeutic effect. As a matter of fact it is well known that CDPC, when orally administered by means of hitherto known pharmaceutical compositions, exposes itself to a progressive enzymatic degradation in the gastrointestinal pathway.

It was now surprisingly found that the above mentioned limit can be obviated and CDPC hematic levels which are therapeutically effective are obtainable when utilizing the pharmaceutical compositions which are object of the present invention, said compositions having the active ingredient carried by means of liposomes.

Pharmaceutical compositions in which the active ingredient is carried by means of liposomes are pharmaceutical compositions in which the active ingredient is included, or dispersed or variously present in globules which are formed by concentric aqueous layers spaced and held by lipidic layers (hydrophobic); said globules, which have a diameter which generally ranges from 15 nm and 5 $\mu$, are generally dispersed in an aqueous phase, in that way forming a dishomogeneous system which is known as liposomic suspension. In order to increase its stability, liposomic suspension can be lyophilized, therefore resulting a dry powder.

The hydrophobic phase is advantageously, but not exclusively, formed by phospholipids, such as lecithine, sphingomyelins and phosphatidylcholine from eggs or soybean; it must be noticed however, that carrying action of liposomes does not depend upon the chemical nature of the hydrophobic phase, rather upon the physical structure of liposomes, as already extensively described in relevant literature; said physical structure allows, either owing to phenomena of inclusion of liposomes into the cells, or owing to phenomena of fusion of liposomes with some components of the cell-membranes, an easy penetration into the cells of pharmaceutically active substances. For example, said endocytosis possibility, due to physical structure of liposomes, significantly increases, when the active ingredient is carried by means of liposomes, absorption levels of the active ingredient in comparison with situations in which same active ingredient is simply in association with phospholipids.

According to the known art, for the preparation of

- 3 -

pharmaceutical compositions in which the active ingredient is carried by means of liposomes, it is generally utilized a process which comprises to form liposomes directly in solutions which hold active ingredients by means of suitable mechanical treatments: for example solutions are sonicated.

However that way of operating has manifest limits when utilized with thermolabile active ingredients, which are submitted to considerable degradation owing to the heat produced during sonication; moreover, one met till now with remarkable difficulties when trying to include substantial amount of active ingredients into liposomes. It was now surprisingly found, and this is a further object of the present invention, that pharmaceutical compositions for oral administration of CDPC, which are object of the present invention can be prepared by means of a process characterized in that the solution which contains the active ingredient is added to globules of hydrophobic phase which were already obtained and lyophilized. Liposomes obtained after adding the solution containing the active ingredient can be subsequently lyophilized; this is still further object of the present invention. The dry powder which is obtained in that way can be utilized for preparing various pharmaceutical preparations such as powders for reconstitution of extemporaneous suspensions, capsules, small-envelopes and tablets.

Globules of the hydrophobic phase can be prepared, and this is still further object of the present invention, by means of a process characterized in that the component of the hydrophobic phase is dissolved in a suitable solvent, is precipitated by adding acetone is separated and then is suspended in water and lyophilized.

Component of the hydrophobic phase is advantageously a phospholipid, and, usefully, phosphatidylcholine; it can be dissolved in a suitable solvent, advantageously ethyl ether.

After adding acetone it is useful to cool in order to help the precipitation. The precipitate can be separated by decantation as well as by filtration or by centrifugation. Water is added to the separed solid; then one proceeds to lyophilize.

Moreover, and this is still further object of the present invention, the component of the hydrophobic phase can be dissolved in a suitable organic solvent, then dispersed in water, to obtain a dispersion which is submitted firstly to evaporation of the organic solvent, later to lyophilization. The organic solvent can be advantageously selected in the group consisting of chloroform, ethyl ether, petroleum ether and ethanol.

Lastly, globules of the component of the hydrophobic phase can be prepared dissolving said component, advantageously a phospholipid, for example phosphatidylcholine from eggs or soybean, in a suitable organic solvent, advantageously chloroform. Said solvent is evaporated and the residue is dispersed in water by sonication, preferably keeping cool the solution. Then one lyophilizes.

To globules of the component of the hydrophobic phase, which are obtained as hereinbefore described, the concentrated aqueous solution of CDPC is added. In that way, substantially higher amounts of the active ingredient than in the prior art can be included into liposomes. If the addition of the solution of the active ingredient is carried out immediately, it is appropriate to provide for a subsequent lyophilization, preferably into the final container.

Otherwise, the addition of the solution of the active ingredient can be carried out at the time of use; in that case the lyophilization of globules of the hydrophobic phase can be carried out in the final container, which can be equipped, for instance, with a suitable tank-cap containing water and with a container-cap containing the active ingredient: the reconstitution of the liposomic suspension is carried out at the time of use.

In the pharmaceutical compositions which are object of the present invention, the hydrophobic phase of liposomes is advantageously formed by phosphatidylcholine, which is utilized in a ratio with CDPC ranging from phosphatidylcholine : CDPC 1 : 1 to 20 : 1. Advantageously said ratio ranges from 1: 1 to 8 : 1.

The pharmaceutical compositions which are object of the present invention can appear as ready-to-use liposomic suspensions, but, preferably, they appear as dry powders for extemporaneous suspensions to be reconstituted with water or as capsules or tablets. Capsules and tablets can be prepared utilizing methods and excipients which are well known in the art and extensively described, for example, in "Pharmaceutical technology" of S. Casadio, Ed. Cisalpino Goliardica - Milano.

Examples of excipients which tablets and capsules may contain are excipients such as binding agents, for example syrup, acacia, gelatin, sorbitol, traganth or polyvinylpirrolidone; fillers, for example lactose, sugar, maize-starch, calcium phosphate, sorbitol, levilite or glycine; lubricants, for example magnesium stearate, talc, polyethylene glycol, silica; disintegrants, for example potato starch or acceptable wetting agents such as sodium lauryl sulphate.

Examples of excipients which dry powders for extemporaneous suspension may contain are excipients such as, for example, lactose or calcium phosphate.

The tablets may be coated according to methods well known in the art. Examples of tablets compositions are given in following Tables A and B.

Table A

| Composition of one single tablet | | |
|---|---|---|
| CDPC sodium salt | mg | 156.76 |
| Phosphatidylcholine | mg | 600 |
| Glycine | mg | 199.5 |
| Levilite | mg | 50 |
| Magnesium stearate | mg | 50 |
| Talc | mg | 50 |
| Calcium phosphate dihydrate | mg | 867 |

Table B

| Composition of one single tablet | | |
|---|---|---|
| CDPC sodium salt | mg | 105.50 |
| Phosphatidylcholine | mg | 400 |
| Glycine | mg | 133 |
| Levilite | mg | 50 |
| Magnesium stearate | mg | 50 |
| Talc | mg | 50 |
| Calcium phosphate dihydrate | mg | 867 |

The dosage of CDPC to be administered, which have to be a therapeutically effective amount, depends to a large extent upon the general health and weight of the subject being treated. In general, a daily dosage consists of from about 500 to about 1800 mg of CDPC in one or more application per day. A preferred daily dosage for adult humans lies in the range of from about 1000 to 1200 mg of active ingredient. The pharmaceutical preparations will generally contain from about 100 mg to about 1 g of CDPC; however, in general, it is preferably to employ a dosage amount in the range of from 100 mg to 200 mg.

- 8 -

It is known that therapeutical and pharmaceutical properties of CDPC include stimulating action of cerebral circulation, antithrombogenetic action and capillary vessels protective action.

The following Examples illustrate some embodiments of the present invention, without limiting it in any way.

EXAMPLE No. 1

10 g of purified phosphatidylcholine from eggs are weighed and dissolved into 50 ml of chloroform. The solution is transferred into a suitable vessel and the solvent is evaporated off under vacuum in order to obtain a thin layer of phospholipids. 50 ml of distilled water are added and the system is kept under stirring enough time to allow the separation of phospholipids from the vessel and the formation of multilayer liposomes.

Then the system is submitted to sonication, while it is kept cold, in order to obtain liposomes having a diameter below 1 $\mu$. Then the suspension is frozen at the temperature of - 50°C and it is lyophilized. 10 ml of 25% cytidine diphosphate choline ester solution are added to the lyophilized matter and a further lyophilization is carried out.

Ratio phosphatidylcholine: cytidine diphosphate choline ester 1 : 1.

EXAMPLE No. 2

One operates as described in Example No. 1, but employing the ratio phosphatidylcholine : cytidine diphosphate

choline ester 2 : 1 and purified phosphatidylcholine from soybean.

EXAMPLE No. 3

One operates as described in Example No. 1, but employing the ratio phosphatidylcholine : cytidine diphosphate choline ester 4 : 1.

EXAMPLE No. 4

One operates as described in Example No. 1, but employing the ratio phosphatidylcholine : cytidine diphosphate choline ester 8 : 1.

EXAMPLE No. 5

One operates as described in Example No. 1, but employing the ratio phosphatidylcholine : cytidine diphosphate choline ester 20 : 1.

EXAMPLE No. 6

10 g of purified phosphatidylcholine from soybean are weighed and dissolved in 50 ml of absolute ethanol; the obtained solution is injected, under pressure and by means of a fine nozzle, into 100 ml of distilled water. Size of obtained liposomes is function of the applied pressure and of the nozzle's diameter. The obtained suspension is concentrated under nitrogen at a temperature < 45°C up to the volume of about 50 ml; then it is lyophilized. 10 ml of a 25% solution of cytidine diphosphate choline ester are added to the lyophilized matter and a further lyophilization is carried out.

EXAMPLE No. 7

0114577

- 10 -

10 g of purified phosphatidylcholine from soybean are weighed and dissolved in 30 ml of peroxides-free ethyl ether; 300 ml of acetone are added while keeping the solution under stirring. A soft precipitate is obtained; its amount increases in consequence of cooling of the suspension to - 20°C for a time from 1 to 2 hours. The solid portion is separated by means of centrifugation. 50 ml of water are added to the separated solid, which is allowed to swell up, up to formation of liposomes which are tiny enough. Then lyophilization is carried out; subsequently 10 ml of 25% solution of the active ingredient are added, as described in Example No. 6.

EXAMPLE No. 8

Composition obtained as described in Example No. 1 is blended with one percent magnesium stearate and filled into size 0 hard gelatin capsules, each capsule containing a claimed dose of 100-200 mg of CDPC. The capsules are packed in glass vials with plastic caps giving moisture-proof seal.

Surprising results which are obtained orally administering carried-by-liposomes CDPC instead of utilizing hitherto known pharmaceutical compositions for oral administration are confirmed by data obtained by carrying out tests hereinbelow described. During the tests as pharmaceutical composition containing carried-by-liposomes CDPC, CDPC carried by means of a liposome having purified phosphatidylcholine from soybean as hydrophobic phase was utilized; said pharmaceutical composition will be called hereinafter Lip. CDPC for the sake of brevity. Moreover, during the hereinbelow describ ed tests, a marked CDPC was utilized having an atom of $^{14}$C introduced into the methyl group of the cholinic side of the molecule. The radioactivity resulted to be about 0.1 μ Curie/mg pure CDPC.

The suspension of Lip. CDPC containing $^{14}$C-CDPC, which was obtained as described in Example No. 1, and a reference aqueous solution of $^{14}$C-CDPC were administered by gavage to rats of average weight of 200 g at the doses of 200 mg/Kg (referred to pure CDPC).

Blood abstractions were carried out at various intervals of time and, 24 hours after the start of test, the animals were sacrificed. Amounts of absorbed active ingredient were calculated from radioactivity measures on serum; said amounts, as reported in following Tables (1 - 2), were higher on the average in tests carried out employing Lip. CDPC in comparison with controls with pure CDPC in aqueous solution. It is apparent from the analysis of Tables that, in the described experimental conditions, there is a valuable absorption of CDPC after oral administration. The amount of said absorption and pertinent hematic concentrations are remarkably higher when Lip. CDPC, which is object of the present invention, is used: for example at the 6th hour the serum concentration of CDPC for Lip. CDPC was much higher than concentration found for CDPC administered in aqueous solution.

- 12 -

## Table No. 1

Serum concentration CPM x $10^{-3}$

| Hours | CDPC | Lip. CDPC |
|---|---|---|
| 0 | 0 | 0 |
| 1 | 0.3 | 1 |
| 2 | 1 | 3 |
| 5 | 2.6 | 7 |
| 10 | 4 | 11 |
| 15 | 4 | 13.4 |

## Table No. 2

Serum concentration DPM/ml

| Time | CDPC 25 mg/Kg (intra venous administration) | CDPC 200 mg/Kg (oral administration) | Lip.CDPC 200 mg/Kg (oral administration) |
|---|---|---|---|
| 5' | 10500 | – | – |
| 15' | 4000 | – | – |
| 30' | 1700 | 50 | 110 |
| 45' | 1500 | 47 | 110 |
| 60' | 1480 | 60 | 120 |
| 2 h | 2100 | 75 | 130 |
| 3 h | 2300 | 170 | 190 |
| 4 h | 2500 | 200 | 1100 |
| 6 h | 2700 | 470 | 2500 |
| 8 h | 2600 | 850 | 3530 |
| 10 h | 2500 | 750 | 3100 |
| 15 h | 1900 | 630 | 3200 |
| 24 h | 1750 | 322 | 433 |

- 13 -

Moreover, a comparison of respective AUC $_0^{30\ h}$ values shows a bioavailability for Lip. CDPC which is 2-3 times higher than for CDPC in aqueous solution when administered orally and only slightly lower than for an intra-venous administration.

Therefore, comparative pharmacological tests carried out on animals showed that pharmaceutical compositions, which are object of the present invention, when orally administered show a good bioavailability and therefore their use gives undoubted advantages as it allows to obtain satisfactory therapeutic results which are comparable with results which are obtained by means of usual CDPC aqueous solutions administered intra-venously while it is utilized an administration way which is more practical and preferred by patients.

While the invention was described in detail and referring to specific embodiments, it is apparent to those skilled in the art that variations and modifications can be made without departing from the scope of the invention.

- 14 -

C L A I M S

1) Pharmaceutical composition for oral administration of cytidine diphosphate choline ester or its salts, characterized in that the active ingredient is carried by means of liposomes.

2) Pharmaceutical composition according to claim 1, characterized in that the hydrophobic phase of liposomes is formed by phosphatidylcholine.

3) Pharmaceutical composition according to claim 2, characterized in that the ratio phosphatidilcholine : cytidine diphosphate choline ester ranges from 1 : 1 to 20 : 1.

4) Process for the preparation of a pharmaceutical composition according to any of claims from 1 to 3, characterized in that the solution containing the cytidine diphosphate choline ester is added to globules of the hydrophobic phase previously obtained and lyophilized.

5) Process according to claim 4, characterized in that said globules of the hydrophobic phase are obtained dissolving the component of the hydrophobic phase in a suitable solvent, precipitating by adding acetone, separating the precipitate, dispersing same in water and lyophilizing.

6) Process according to claim 4, characterized in that said globules of the hydrophobic phase are obtained dissolving the component of the hydrophobic phase in a suitable organic solvent, dispersing in water, evaporating the organic solvent and lyophilizing.

7) Pharmaceutical composition for oral administration of cytidine diphosphate choline ester, characterized in that it is prepared as substantially herein described with particular reference to Examples 1, 2, 3, 4, 5, 6 and 7.

European Patent Office

**EUROPEAN SEARCH REPORT**

0114577
Application number

EP 83 83 0007

## DOCUMENTS CONSIDERED TO BE RELEVANT

| Category | Citation of document with indication, where appropriate, of relevant passages | Relevant to claim | CLASSIFICATION OF THE APPLICATION (Int. Cl. 3) |
|---|---|---|---|
| A | CHEMICAL ABSTRACTS, vol. 96, no. 20, 17th May 1982, page 393, no. 168619q, Columbus, Ohio, USA R.J. PARKER et al.: "Comparison of lymphatic uptake, metabolism, excretion, and biodistribution of free and liposome-entrapped [14C]cytosine beta-D-arabinofuranoside following intraperitoneal administration to rats" & DRUG METAB. DISPOS. 1982, 10(1), 40-46 * Abstract * | 1 | A 61 K 9/50 |
| A | GB-A-2 074 445 (MADE ITALIANA SRL) * Column 1, paragraphs 1-3,7; claims * | 1,3 | |

TECHNICAL FIELDS SEARCHED (Int. Cl. 3)

A 61 K 9/00
C 07 H 19/00
A 61 K 31/00

The present search report has been drawn up for all claims

| Place of search THE HAGUE | Date of completion of the search 22-08-1983 | Examiner BERTE M.J. |
|---|---|---|